# FASCICULE DE BREVET EUROPEEN

(11) **EP 1 591 103 B1**
(45) Date de publication et mention de la délivrance du brevet: **02.06.2010**
(21) Numéro de dépôt: 05290773.0
(22) Date de dépôt: 07.04.2005
(51) Int. Cl.: A61K 8/81, A61K 8/60, A61Q 5/00

(54) **Composition détergente comprenant un tensioactif non ionique et un polymère anionique et utlisation pour protéger la couleur**
Einen nichtionischen Emulgator und ein anionisches Polymer enthaltende Waschmittelzusammensetzung und deren Verwendung um die Farbe zu schützen
Detergent composition comprising a nonionic surfactant and an anionic polymer; use of said composition for preserving colour

(30) Priorité: 30.04.2004 FR 0404650
(43) Date de publication de la demande: 02.11.2005
(73) Titulaire: L'Oréal, 75008 Paris (FR)
(72) Inventeur: Lalleman, Boris, 75015 Paris (FR); Giroud, Franck, 92110 Clichy (FR)
(74) Mandataire: Le Blainvaux Bellegarde, Françoise

(56) Documents cités:
- EP-A- 0 502 616
- EP-A- 0 617 954
- EP-A- 0 739 619
- EP-A- 1 142 556
- EP-A- 1 342 462
- WO-A-01/76552
- NOVEON: "Ultra Mild Conditioning Shampoo with Suspended Mica Using Carbopol ETD 2020 Polymer" INTERNET ARTICLE, 28 novembre 2000 (2000-11-28), XP002305256 Extrait de l'Internet: URL:http://www.personalcare.noveoninc.com/ formulations/EU0010.pdf> [extrait le 2004-11-11]
- NOVEON: "2-In-1 Conditioning Shampoo Using Carbopol ETD 2020 Polymer" INTERNET ARTICLE, 24 mars 1999 (1999-03-24), XP002305257 Extrait de l'Internet: URL:http://www.personalcare.noveoninc.com/ formulations/EU0004.pdf> [extrait le 2004-11-11]
- NOVEON: "Carbopol Aqua SF-1 Polymer Clear Baby Shampoo Formulation" INTERNET ARTICLE, [Online] décembre 2000 (2000-12), XP002305255 Extrait de l'Internet: URL:http://www.personalcare.noveoninc.com/ formulations/CASF1-021.pdf> [extrait le 2004-11-11]
- KARHE J ET AL: "ALKYLPOLYGLYCOSIDE EIN NEUES KONZEPT FUR PFLEGE UND VERTRAGLICHKEIT IN DER KOSMETIK" SOFW-JOURNAL SEIFEN, OELE, FETTE, WACHSE, VERLAG FUR CHEMISCHE INDUSTRIE, H. ZIOLKOWSKY K.G. AUGSBURG, DE, vol. 121, no. 8, 1 juillet 1995 (1995-07-01), pages 598,600-601,604, XP000514057 ISSN: 0942-7694

## Description

La présente invention a pour objet une composition telle que définie dans la revendication 1 comprenant un polymère anionique et un tensioactif non ionique, ou leurs mélanges, un procédé de traitement de fibres kératiniques notamment colorées tel que défini dans la revendication 24 ainsi que l'utilisation de la composition pour protéger la coloration desdites fibres telle que définie dans la revendication 23.

Plus précisément, le domaine de l'invention est celui du traitement des fibres kératiniques notamment colorées, de préférence de fibres kératiniques humaines, comme notamment les cheveux.

Pour le nettoyage et/ou le lavage des cheveux, l'utilisation de compositions détergentes (ou shampooings) à base essentiellement d'agents tensioactifs classiques de type notamment anionique est courante. Ces compositions sont appliquées sur cheveux mouillés et la mousse générée par massage ou friction avec les mains permet, après rinçage à l'eau, l'élimination des diverses salissures initialement présentes sur les cheveux.

Ces compositions de base possèdent certes un bon pouvoir lavant, mais les propriétés cosmétiques intrinsèques qui leur sont attachées restent toutefois assez faibles, notamment en raison du fait que le caractère relativement agressif d'un tel traitement de nettoyage peut entraîner à la longue sur la fibre capillaire des dommages plus ou moins marqués liés en particulier à l'élimination progressive des lipides ou protéines contenues dans ou à la surface de cette dernière.
De plus sur cheveux colorés, les shampoings entraînent un phénomène de dégorgement de la coloration non négligeable.

Les agents tensioactifs de la famille des alkylpolyglycosides ou polyglycérolés ont déjà été préconisés dans des compositions lavantes pour les cheveux ou la peau. Ce sont des détergents doux, bien tolérés et biodégradables.

EP-0 617 954-A1 décrit une composition cosmétique comprenant, dans un milieu cosmétiquement acceptable, au moins un agent tensio-actif non ionique de la famille des alkylpolyglycosides et/ou des tensio-actifs polyglycérolés et au moins un copolymère réticulé d'anhydride maléique/alkyl (C₁-C₅) vinyléther.

Généralement, les compositions lavantes sont épaissies. On recherche des produits que l'on peut doser et prendre aisément dans la main. Pour ce faire, ces produits doivent présenter une certaine consistance ou viscosité. En effet, un produit liquide est beaucoup plus difficile à doser et s'écoule facilement entre les doigts, ce qui gêne l'application sur les cheveux. Par ailleurs, dans le cas d'un shampooing, le produit peut s'écouler sur le visage et en particulier dans les yeux, ce qui n'est pas souhaitable.

Cependant, l'épaississement des compositions à base de tensioactifs non ioniques est difficilement réalisé par les méthodes traditionnelles (amides de coprah, électrolytes..) en raison de leur structure non-ionique. Pour épaissir les compositions cosmétiques contenant ces agents tensioactifs, on a déjà utilisé des polymères synthétiques ou naturels tels que les polymères acryliques réticulés du type Carbopol, les celluloses... Malheureusement, la plupart des épaississants donnent des compositions instables et/ou non transparentes. De plus, certains agents épaississants, notamment les épaississants associatifs à chaînes grasses, présentent l'inconvénient de diminuer la qualité de la mousse et les performances cosmétiques des shampooings, notamment en rendant les cheveux plus chargés et plus rêches. La mousse de des compositions épaissies n'est généralement pas suffisamment douce, elle ne se développe pas facilement que ce soit en vitesse ou en abondance.

Ainsi, il subsiste le besoin d'un système épaississant permettant d'épaissir, de manière convenable une composition lavante comprenant des tensioactifs non ioniques sans influer sur les propriétés cosmétiques et moussantes desdites compositions.

Le but de la présente invention est d'obtenir des compositions détergentes ne dégradant pas la couleur (coloration) des cheveux, ayant une bonne viscosité et donnant une mousse abondante, fine, et se développant facilement.

La demanderesse vient de découvrir, de manière surprenante, que l'association, dans des compositions lavantes pour matières kératiniques, de copolymères anioniques acryliques particuliers à des agents tensioactifs non ioniques, notamment de type alkylpolyglycoside et/ou polyglycérolé permettait d'obtenir des compositions épaissies, stables, homogènes et limpides. Par ailleurs ces compositions permettent d'obtenir une mousse très douce Le démarrage de la mousse est rapide et la mousse est stable et se rince facilement.

Enfin, la composition lavante permet une bonne protection de la coloration. Elle permet en effet un moindre dégorgement induisant un moindre décapage de la coloration au fur et à mesure des shampooings.
On a aussi constaté, par voie de conséquence, que le moindre dégorgement du ou des colorants se traduisait par un risque de tâchage de la peau et des tissus moins important.

Ainsi, la présente invention a pour objet une composition cosmétique détergente comprenant dans un milieu aqueux notamment physiologiquement acceptable et en particulier cosmétiquement acceptable, de 5 à 20% en poids d'au moins un tensioactif non ionique choisi parmi :
- les alkylpolyglycosides ;
et au moins 0,01 à 10% en poids par rapport au poids total de la composition d'un copolymère anioniques réticulé d'acide carboxylique vinylique (monomère A) et d'un monomère ayant au moins une insaturation a,β-éthylénique (monomère B) choisis parmi les monomères de formules :
i) CH₂=CXY
dans laquelle X désigne l'hydrogène et Y désigne -COOR, -C6H4R', -CN, -CONH₂, -NHCOCH₃, -CONHC(CH₃)₃, -CON(CH₃)₂, ou
X désigne CH₃ et Y désigne -COOR, -C₆H₄R', -CN ou -CH=CH₂,
R désigne un alkyle en C₁-C₄ ou un hydroxyalkyle en C₂-C₄,
R' désigne hydrogène, un alkyle en C₁-C₈ ou un hydroxyalkyle en C₂-C₈.

Elle a enfin pour objet l'utilisation d'une composition telle que définie ci-dessus pour la limitation du dégorgement de la coloration et/ou la protection de la coloration des fibres kératiniques.

La présente invention a également pour objet de proposer un procédé de traitement des fibres kératiniques colorées permettant d'avoir un phénomène limité de dégorgement de la couleur lors des shampooings tout en bénéficiant de bonnes qualités d'usages pour lesdits shampooings.

Un autre objet de l'invention est constitué par le procédé de lavage et/ou de conditionnement mettant en oeuvre de telles compositions.

Mais d'autres avantages et caractéristiques de l'invention apparaîtront plus clairement à la lecture de la description et des exemples qui vont suivre.

Le copolymère comprend un ou plusieurs monomères A, un ou plusieurs monomères B et un ou plusieurs agent réticulant.

Les acides carboxyliques vinyliques (monomères A) sont notamment choisis parmi l'acide acrylique, l'acide méthacrylique, l'acide itaconique, l'acide fumarique, l'acide crotonique, l'acide maléique et leurs mélanges. Les acides carboxyliques vinyliques sont de préférence choisis parmi l'acide acrylique et l'acide méthacrylique, et plus particulièrement l'acide méthacrylique.

Les monomère B ayant au moins une insaturation a,β-éthylénique sont choisis parmi les monomères de formule :
i) CH₂=CXY
dans laquelle X désigne l'hydrogène et Y désigne -COOR, -C6H4R', -CN, -CONH₂, -NHCOCH₃, -CONHC(CH₃)₃, -CON(CH₃)₂, ou
X désigne CH₃ et Y désigne -COOR, -C₆H₄R', -CN ou -CH=CH₂,
R désigne un alkyle en C₁-C₄ ou un hydroxyalkyle en C₂-C₄,
R' désigne hydrogène, un alkyle en C₁-C₈ ou un hydroxyalkyle en C₂-C₈.

Parmi ces monomères B, on peut citer l'acrylate de méthyle, l'acrylate d'éthyle, l'acrylate de n-butyle, l'acrylate de 2-hydroxyéthyle, le styrène, l'acrylamide, le N,N-diméthylacrylamide, le tertio-butylacrylamide, le méthacrylate de méthyle, le méthacrylate d'éthyle, le méthacrylate de n-butyle, le méthacrylate de 2-hydroxyéthyle.

Le monomère B ayant au moins une insaturation α,β-éthylénique est un monomère de formule i) dans laquelle X désigne l'hydrogène et Y désigne -COOR. Plus particulièrement, le monomère B est choisi notamment parmi l'acrylate de méthyle, l'acrylate d'éthyle et l'acrylate de butyle et plus particulièrement l'acrylate d'éthyle.

Le monomère A est présent de préférence dans des quantités allant de 20 à 80% en poids et plus particulièrement de 25 à 70 % en poids et encore plus particulièrement de 35 à 60% en poids par rapport au poids total du copolymère.

Le monomère B est présent de préférence dans des quantités allant de 15 à 80% en poids et plus particulièrement de 25 à 75 % en poids et encore plus particulièrement de 40 à 65% en poids par rapport au poids total du copolymère.

Ce copolymère est partiellement ou totalement réticulé par au moins un agent réticulant classique. Les agents réticulants sont notamment des composés polyinsaturés, en particulier éthylèniquement polyinsaturés. Ces composés sont notamment les polyalcényléthers de sucrose ou de polyols, les diallylphtalates, le divinylbenzène, le (méth)acrylate d'allyle, di(méth)acrylate d'éthylèneglycol, le méthylène bis-acrylamide, le tri(méth)acrylate de triméthylol propane, l'itaconate de diallyle, le fumarate de diallyle, le maléate de diallyle, le (méth)acrylate de zinc, les dérivés d'huile de ricin ou de polyols fabriqués à partir d'acides carboxyliques insaturés.
Comme agent réticulant, on peut également utiliser des composés monomères insaturés et comportant un groupe réactif susceptible de réagir avec une insaturation pour former un copolymère réticulé.

La teneur en agent réticulant varie en général de 0,01 à 5% en poids et de préférence de 0,03 à 3% en poids et encore plus particulièrement de 0,05 à 1 % en poids par rapport au poids total du copolymère.

Le copolymère préféré selon l'invention est choisi parmi un copolymère réticulé d'acide méthacrylique et d'acrylate d'alkyle en C₁-C₄ , un copolymère réticulé d'acide acrylique et d'acrylate d'alkyle en C₁-C₄ et plus particulièrement un copolymère réticulé d'acide méthacrylique et d'acrylate d'éthyle .

Selon une forme particulièrement préférée, le copolymère de l'invention peut se présenter notamment sous forme de dispersion dans l'eau. La taille moyenne en nombre des particules de copolymère dans la dispersion est généralement comprise entre 10 et 500 nm et de préférence entre 20 et 200 nm et plus préférentiellement de 50 à 150 nm.

Ces copolymères sont notamment décrit dans la demande WO01/76552.

On utilisera plus particulièrement un copolymère réticulé acide méthacrylique/acrylate d'éthyle sous forme de dispersion aqueuse à 30% fabriqué et vendu sous le nom de CARBOPOL AQUA SF-1 par la société NOVEON ou encore un copolymère réticulé d'acide (méth)acrylique et d'acrylate d'alkyle en C1-C4 vendu sous la dénomination ACULYN 33 par la société ROHM&HAAS.

La concentration en copolymère réticulé va généralement de 0,01 à 10% en poids par rapport au poids total de la composition et de préférence de 0,1 à 5% en poids et encore plus particulièrement de 0,5 à 3% en poids.

La composition détergente comprend à titre de tensioactif, au moins un tensioactif non ionique choisi parmi :
- les alkylpolyglycosides.

La composition lavante comprend au moins un tensioactif non ionique choisi parmi les alkylpolyglycosides.

En ce qui concerne les alkypolyglycosides, ces composés sont bien connus et peuvent être plus particulièrement représenté par la formule générale suivante :

R₁O-(R₂O)ₜ (G)ᵥ (II)

dans laquelle R₁ représente un radical alkyle et/ou alcényle linéaire ou ramifié comportant environ de 8 à 24 atomes de carbone, un radical alkylphényle dont le radical alkyle linéaire ou ramifié comporte de 8 à 24 atomes de carbone, R₂ représente un radical alkylène comportant environ de 2 à 4 atomes de carbone, G représente un motif sucre comportant de 5 à 6 atomes de carbone, t désigne une valeur allant de 0 à 10, de préférence 0 à 4, de préférence 0 à 4 et v désigne une valeur allant de 1 à 15.

Des alkylpolyglycosides préférés selon la présente invention sont des composés de formule (II) dans laquelle R₁ désigne plus particulièrement un radical alkyle saturé ou insaturé, linéaire ou ramifié comportant de 8 à 18 atomes de carbone, t désigne une valeur allant de 0 à 3 et plus particulièrement encore égale à 0, G peut désigner le glucose, le fructose ou le galactose, de préférence le glucose. Le degré de polymérisation, i.e. la valeur de v dans la formule (II), peut aller de 1 à 15, de préférence de 1 à 4. Le degré moyen de polymérisation est plus particulièrement compris entre 1 et 2 et encore plus préférenciellement de 1,1 à 1,5.
Les liaisons glycosidiques entre les motifs sucre sont de type 1-6 ou 1-4 et de préférence 1-4.

Des composés de formule (II) sont notamment représentés par les produits vendus par la société COGNIS sous les dénominations PLANTAREN^{®} (600 CS/U, 1200 et 2000) ou PLANTACARE^{®} (818, 1200 et 2000). On peut également utiliser les produits vendus par la société SEPPIC sous les dénominations TRITON CG 110 (ou ORAMIX CG 110) et TRITON CG 312 (ou ORAMIX^{®} NS 10), les produits vendus par la société B.A.S.F. sous la dénomination LUTENSOL GD 70 ou encore ceux vendus par la société CHEM Y sous la dénomination AG10 LK.
On peut également utiliser par exemple, l'Alkyl en C8/C16 polyglucoside 1,4 en solution aqueuse à 53% commercialisé par COGNIS sous la référence PLANTACARE^{®} 818 UP.

Le ou les tensioactifs non ioniques sont présents dans les compositions selon l'invention dans des concentrations allant de 5 à 250 % en poids, de préférence de 8 à 18% en poids par rapport au poids total de la composition.

La composition selon l'invention peut comprendre en outre d'autres tensioactifs tels que des tensioactifs anioniques, amphotères, zwittérioniques et leurs mélanges.

Pour ce qui a trait aux tensioactifs amphotères ou zwittérioniques, on peut mentionner sans avoir l'intention de s'y limiter, les dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) betaïnes ou les alkyl (C₈-C₂₀) amidoalkyl (C₁-C₆) sulfobétaïnes.

Parmi les dérivés d'amines, on peut citer les produits vendus sous la dénomination MIRANOL, tels que décrits dans les brevets US-2 528 378 et US-2 781 354 et classés dans le dictionnaire CTFA, 3ème édition, 1982, sous les dénominations Amphocarboxyglycinates et Amphocarboxypropionates de structures respectives : R₂ -CONHCH₂CH₂ -N(R₃)(R₄)(CH₂COO-)
dans laquelle : R₂ désigne un radical alkyle d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle ;
et
R₂'-CONHCH₂CH₂-N(B)(C)
dans laquelle :
B représente -CH₂CH₂OX', C représente -(CH₂)_{z}-Y', avec z = 1 ou 2,
X' désigne le groupement -CH₂CH₂-COOH ou un atome d'hydrogène
Y' désigne -COOH ou le radical -CH₂-CHOH-SO₃H
R₂' désigne un radical alkyle d'un acide R₉-COOH présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylampho-dipropionate, Disodium Capryloamphodipropionate, Lauroamphodipropionic acid, Cocoamphodipropionic acid, disodium cocoamphocarboxy ethyl hydroxypropyl sulfonate.
A titre d'exemple on peut citer le cocoamphodiacetate commercialisé sous la dénomination commerciale MIRANOL^{®} C2M concentré par la société Rhodia Chimie.

Les tensioactifs anioniques éventuellement présents sont de préférence des tensioactifs anioniques doux.

En ce qui concerne les tensioactifs anioniques doux, on peut citer notamment les composés suivants et leurs sels, ainsi que leurs mélanges :
- les acides alkyl éther carboxyliques polyoxyalkylénés,
- les acides alkylaryl éther carboxyliques polyoxyalkylénés,
- les acides alkylamido éther carboxyliques polyoxyalkylénés en particulier ceux comportant 2 à 50 groupements oxyde d'éthylène,
- les acides d'alkyl D galactoside uroniques
- les acylsarcosinates, les acylglutamates ;
- les esters d'alkylpolyglycosides carboxyliques;

Tout particulièrement, on utilise des acides alkyl éther carboxyliques polyoxyalkylénés comme par exemple l'acide lauryl ether carboxylique (4,5 OE) commercialisé par exemple sous la dénomination AKYPO RLM 45 CA de KAO.

Si de tels tensioactifs additionnels sont présents, alors la teneur en tensioactif(s) additionnel(s) va de 0,1 à 20% en poids, par rapport au poids total de la composition lavante, plus particulièrement de 1 % à 10 % en poids, par rapport au poids total de la composition.

De préférence, la composition lavante ne contient pas de tensioactif détergent anionique de type sulfate ( alkylsulfate ou alkylether sulfate, alkylamidoether sulfate). Et si elle en contient, sa teneur est telle que le rapport pondéré : tensioactif détergent anionique de type alkylsulfate ou alkylether sulfate / somme des tensioactifs amphotère, zwittérionique, anionique doux et non ionique soit de préférence inférieur ou égal à 1, et plus particulièrement inférieur ou égal à 0,75 et encore plus préférentiellement inférieur ou égal à 0,5.

La composition lavante peut de plus comprendre les additifs classiques dans le domaine comme par exemple ceux choisis parmi la liste non exhaustive tels que les agents réducteurs, les agents oxydants, les séquestrants, les adoucissants, les agents anti-mousse, les agents hydratants, les agents émollients, les agents alcalinisants, les plastifiants, les filtres solaires, les colorants directs ou d'oxydation, les pigments, les charges minérales, les argiles, les minéraux colloïdaux, les nacres, les agents nacrant, les parfums, les peptisants, les conservateurs, les polymères fixant ou non, les protéines, les vitamines, les agents antipelliculaires, les agents antiséborrhéïques, les agents antichute des cheveux, les alcools aliphatiques ou aromatiques, et plus particulièrement l'éthanol, l'alcool benzylique, les polyols modifiés ou non tels que le glycérol, le glycol, le propylène glycol, le dipropylène glycol, le butylène glycol, le butyle diglycol, les silicones volatiles, les huiles minérales, organiques ou végétales, les cires oxyéthylénées ou non, les paraffines, les acides gras, les polymères épaississants associatifs ou non, les amides grasses, les esters gras, les alcools gras, etc.

Les adjuvants cités ci-dessus sont en général présents en quantité comprise, pour chacun d'eux, entre 0,01 et 20% en poids par rapport au poids de la composition.
Il est à noter que si la composition comprend un ou agents épaississants, leur teneur est comprise entre 0,01 et 20% en poids par rapport au poids de la composition lavante, de préférence de 0,01 à 3% en poids par rapport au poids de la composition lavante.

De préférence, la composition selon l'invention comprend au moins un agent conditionneur.
Lorsque la composition contient au moins un agent de conditionnement, ils sont généralement choisis parmi les huiles de synthèse telles que les poly-α-oléfines, les huiles fluorées, les cires fluorées, les gommes fluorées, les esters d'acides carboxyliques, les polymères cationiques, les silicones, les huiles minérales, végétales ou animales, les céramides, les pseudocéramides et leurs mélanges.

Selon un mode de réalisation préféré, le ou les agents conditionneurs sont choisis parmi les polymères cationiques et/ou les silicones volatile ou non volatile. De préférence l'agent conditionneur est choisi parmi les silicones aminées.

Selon ce mode de réalisation, la teneur en agent conditionneur dans la composition selon l'invention est comprise de 0,001 % à 10 % en poids, de préférence de 0,005 % à 5 % en poids, et encore plus préférentiellement de 0,01 % à 3 % en poids, du poids total de la composition finale.

Parmi les polymères cationiques susceptibles d'être utilisés dans le cadre de la présente invention, on préfère mettre en oeuvre les dérivés d'éther de cellulose quaternaires tels que les produits vendus sous la dénomination JR 400 par la société Amerchol, les cyclopolymères cationiques, en particulier les homopolymères ou copolymères de chlorure de diméthyldiallylammonium, vendus sous les dénominations Merquat® 100, Merquat® 550 et Merquat® S par la société Nalco, les polysaccharides cationiques tels que les gommes de guar modifiées par un sel de 2,3-époxypropyl triméthylammonium, les polymères quaternaires de vinylpyrrolidone et de vinylimidazole et leurs mélanges.

Le milieu aqueux notamment physiologiquement acceptable pour les matières kératiniques est généralement constitué par de l'eau ou par un mélange d'eau et d'au moins un solvant organique acceptable sur le plan cosmétique.
A titre de solvant organique, on peut par exemple citer les monoalcools, linéaires ou ramifiés, de préférence saturés, comprenant 2 à 10 atomes de carbone, tels que l'alcool éthylique, l'alcool isopropylique ; les alcools aromatiques tels que l'alcool benzylique, l'alcool phényléthylique ; les polyols ou éthers de polyols tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, le dipropylèneglycol, l'hexylèneglycol (2-méthyl 2,4-pentanediol), le néopentylglycol et le 3-méthyl-1,5-pentanediol ; ainsi que les alkyléthers de diéthylèneglycol, notamment en C₁-C₄, comme par exemple, le monoéthyléther ou le monobutyléther du diéthylèneglycol, seuls ou en mélange.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Selon l'invention, les compositions sont de préférence moussante, c'est-à-dire que le pouvoir moussant des compositions selon l'invention, caractérisé par une hauteur de mousse, est généralement supérieur à 75 mm ; de préférence, supérieure à 100 mm mesurée selon la méthode ROSS-MILES (NF T 73-404 /ISO696) modifiée.
Les modifications de la méthode sont les suivantes :
La mesure se fait à la température de 22°C avec de l'eau osmosée. La concentration de la solution est de 2g/l. La hauteur de la chute est de 1 m. La quantité de composition qui chute est de 200 ml. Ces 200 ml de composition tombe dans une éprouvette ayant un diamètre de 50 mm et contenant 50 ml de la composition à tester. La mesure est faite 5 minutes après l'arrêt de l'écoulement de la composition.
La composition selon l'invention peut contenir un propulseur. Le propulseur est constitué par les gaz comprimés ou liquéfiés usuellement employés pour la préparation de compositions aérosols. On emploiera de manière préférentielle l'air, le gaz carbonique, l'azote comprimé ou encore un gaz soluble tel que le diméthyléther, les hydrocarbures éventuellement halogénés (fluorés en particuliers) ou non et leurs mélanges.

Les compositions peuvent se présenter sous différentes formes galéniques tels qu'une lotion, un gel, un spray, une mousse aérosol, une pompe mousse, etc.

Selon un mode de réalisation de l'invention, la composition lavante est conditionnée dans un dispositif aérosol. Le dispositif comprend alors un propulseur. Le propulseur est choisi de préférence parmi les gaz comprimés ou liquéfiés.

L'invention a encore pour objet un procédé de traitement cosmétique des matières kératiniques telles que les cheveux consistant à appliquer sur celles-ci une composition telle que définie précédemment puis à effectuer éventuellement un rinçage à l'eau, après un éventuel temps de pause.

Des exemples concrets mais non limitatifs de l'invention vont maintenant être présentés.

### EXEMPLE 1

On a préparé les 2 compositions de shampooing suivantes :

| | A | B (comparatif) |
|---|---|---|
| Coco-glucoside à 53% dans l'eau (Plantacare 818 UP-Cognis) | 15gMA | 15gMA |
| Polyquaternium-10 (JR400-Amerchol) | 0.7gMA | 0.7gMA |
| Copolymère réticulé d'acide méthacrylique et d'acrylate d'éthyle à 30% dans l'eau (Carbopol AquaSF-1 de NOVEON) | 2.5gMA | - |
| Acide Polyacrylique réticulé (Carbopol 980 de NOVEON) | - | 2.5gMA |
| HCl qs | pH 6 | pH 6 |
| Eau qsp | 100g | 100g |

La qualité de la mousse des formules a été évaluée sur des mèches de cheveux (vitesse de démarrage, le caractère pas trop aéré, l'abondance et la tenue)

Les conditions d'applications sont les suivantes :
Après avoir été humidifiée par de l'eau (3 passages entre les doigts sous l'eau), chaque mèche est essorée entre deux doigts et une quantité de 0,4 g de shampooing / gramme de cheveu est appliquée le long de la mèche de cheveux ( de la racine à la pointe de façon homogène).

On fait alors mousser en malaxant doucement la mèche entre deux doigts dans sa longueur pendant 15 secondes de haut en bas (sans faire de noeuds).
On observe alors la vitesse de démarrage du shampoing
Puis la mèche est enroulée autour des doigts et disposée dans la coupelle en plastique.

On observe alors l'abondance de mousse et le caractère aéré de chacun des shampooings réalisés.

On laisse alors pauser pendant un temps chronométré à 5 mn pour chaque application et la tenue de la mousse est observée.

| | A | B |
|---|---|---|
| Aspect visuel à 24 h | Gel fluide limpide | Gel opaque granuleux |
| Stabilité 1 semaine à 45°C | stable | Instable (durcit) |

La composition contenant le Carbopol Aqua SF1 (composition A) permet d'obtenir à la fois :
- un aspect esthétique : gel fluide limpide
- une bonne stabilité et possédant une viscosité suffisante pour permettre une application confortable sur la chevelure
- d'excellentes qualités de mousse (vitesse de démarrage de la mousse, caractère aéré de la mousse, abondance et tenue de la mousse)

### EXEMPLE 2

On a préparé les compositions suivantes :

| | C | D (comparatif) |
|---|---|---|
| Coco-glucoside à 53% dans l'eau (Plantacare 818 UP-Cognis) | 15gMA | - |
| Sodium Laureth Sulfate à 25% (TEXAPON N 702) | - | 15gMA |
| Polyquaternium-10 (JR400-Amerchol) | 0.7gMA | 0.7gMA |
| Copolymère d'acide méthacrylique/acrylate d'éthyle réticulé à 30% dans l'eau (Carbopol AquaSF-1 de NOVEON) | 1.8gMA | 1.8gMA |
| HCl qs | pH 6 | pH 6 |
| Eau | qsp 100g | qsp 100 g |
| Aspect à 24 H | Gel fluide homogène transparent | Précipité opalescent |

Le shampooing C contenant une forte quantité de tensio-actif non ionique permet d'obtenir un shampooing d'aspect visuel esthétique (transparent) et stable tandis que le shampooing D avec une forte teneur en TA anionique n'est ni transparent ni stable.

D'autre part, le shampooing C présente un épaississement suffisant, stable, et de bonnes propriétés de mousse, cosmétiques et détergentes.

On constate également sur cheveux colorés avec Majicontrast Rouge que le dégorgement de la couleur dans l'eau et dans la mousse est moins important dans le cas de la présente invention. La ténacité de la coloration est méliorée.

### EXEMPLE 3

On a préparé les compositions suivantes :

| | Shampooing E | Shampooing F |
|---|---|---|
| Coco-glucoside à 53% dans l'eau (Plantacare 818 UP-Cognis) | 15gMA | - |
| POLYGLYCERYL-3 HYDROXYLAURYL ETHER, CHIMEXANE NF de CHIMEX | - | 15gMA |
| Polyquaternium-10 (JR400-Amerchol) | 0.7gMA | 0.7qMA |
| Copolymère d'acide méthacrylique/acrylate d'éthyle réticulé à 30% dans l'eau (Carbopol AquaSF-1 de NOVEON) | 1.8gMA | 1.8gMA |
| HCl qs | pH 6 | pH 6 |
| Eau | Qsp 100 g | Qsp 100 g |
| Aspect à 24 H | Gel fluide transparent | Gel fluide transparent |

Ces compositions donnent toutes satisfaction tant du point de vue de l'aspect que du point de vue de l'application sur les cheveux et des résultats cosmétiques.

### EXEMPLE 4

| | |
|---|---|
| Coco (C8-C16)-glucoside à 53% dans l'eau (Plantacare 818 UP-Cognis) | 15gMA |
| Polyquaternium-10 (JR400-Amerchol) | 0.7gMA |
| Copolymère réticulé d'acide méthacrylique et d'acrylate d'éthyle à 28 % dans l'eau (Aculyn 33 de ROHM&HAAS) | 2 gMA |
| Amino-2 méthyl-2 propanol qs | pH 7 |
| Eau qsp | 100 g |

### EXEMPLE 5

On a préparé les 2 compositions de shampooing suivantes :

| | A | B (comparatif) |
|---|---|---|
| Coco-glucoside à 53% dans l'eau (Plantacare 818 UP-Cognis) | 15gMA | 15gMA |
| Polyquaternium-10 (JR400-Amerchol) | 0.7gMA | 0.7gMA |
| Copolymère réticulé d'acide méthacrylique et d'acrylate d'éthyle à 30% dans l'eau (Carbopol AquaSF-1 de NOVEON) | 2.5gMA | - |
| Copolymère méthylvinyléther/ anhydride maléïque réticulé (Stabileze QM de ISP) | - | 2.5gMA |
| HCl qs | pH 6 | pH 6 |
| Eau qsp | 100 g | 100 g |

La qualité de la mousse des formules a été évaluée sur des mèches de cheveux (vitesse de démarrage, le caractère pas trop aéré, l'abondance et la tenue)

Les conditions d'applications sont les suivantes :
Après avoir été humidifiée par de l'eau (3 passages entre les doigts sous l'eau), chaque mèche est essorée entre deux doigts et une quantité de 0,4 g de shampooing / gramme de cheveu est appliquée le long de la mèche de cheveux (de la racine à la pointe de façon homogène).

On fait alors mousser en malaxant doucement la mèche entre deux doigts dans sa longueur pendant 15 secondes de haut en bas (sans faire de noeuds). On observe alors la vitesse de démarrage du shampoing
Puis la mèche est enroulée autour des doigts et disposée dans la coupelle en plastique.

On observe alors l'abondance de mousse et le caractère aéré de chacun des shampooings réalisés.

On laisse alors pauser pendant un temps chronométré à 5 mn pour chaque application et la tenue de la mousse est observée.

| | A | B |
|---|---|---|
| Aspect visuel à 24 h | Gel fluide limpide | Gel blanc épais inhomogène et granuleux |

La composition contenant le Carbopol Aqua SF1 (composition A) permet d'obtenir à la fois:
- un aspect esthétique : gel fluide limpide
- une bonne stabilité et possédant une viscosité suffisante pour permettre une application confortable sur la chevelure
- d'excellentes qualités de mousse (vitesse de démarrage de la mousse, caractère aéré de la mousse, abondance et tenue de la mousse)

La composition B est un gel épais difficile à répartir sur la chevelure. La mousse démarre moins rapidement, elle est moins abondante.

## Revendications

1. Composition cosmétique détergente comprenant dans un milieu aqueux physiologiquement acceptable et en particulier cosmétiquement acceptable, de 5 à 20% en poids d'un tensioactif non ionique choisi parmi :
• les alkylpolyglycosides ;
et au moins 0,01 à 10% en poids par rapport au poids total de la composition d'un copolymère anioniques réticulé d'acide carboxylique vinylique (monomère A) et d'un monomère ayant au moins une insaturation α,β-éthylénique (monomère B) choisis parmi les monomères de formules :
i) CH₂=CXY
dans laquelle X désigne l'hydrogène et Y désigne -COOR, -C6H4R', -CN, -CONH₂, -NHCOCH₃, -CONHC(CH₃)₃, -CON(CH₃)₂, ou
X désigne CH₃ et Y désigne -COOR, -C₆H₄R', -CN ou -CH=CH₂,
R désigne un alkyle en C₁-C₄ ou un hydroxyalkyle en C₂-C₄,
R' désigne hydrogène, un alkyle en C₁-C₈ ou un hydroxyalkyle en C₂-C₈.

2. Composition selon la revendication 1, **caractérisée en ce que** le monomère B est choisi parmi l'acrylate de méthyle, l'acrylate d'éthyle, l'acrylate de n-butyle, le styrène, l'acrylamide, N,N-diméthylacrylamide, le tertio-butylacrylamide, le méthacrylate de méthyle, le méthacrylate d'éthyle, le méthacrylate de n-butyle.

3. Composition selon la revendication précédente **caractérisée en que** le monomère B est l'acrylate d'éthyle.

4. Composition selon l'une quelconque des revendications 1 à 3, **caractérisée en ce que** le monomère A est choisi parmi l'acide acrylique, l'acide méthacrylique, l'acide itaconique, l'acide fumarique, l'acide crotonique, l'acide maléique et leurs mélanges.

5. Composition selon la revendication précédente **caractérisée en que** le monomère A est choisi parmi l'acide méthacrylique et l'acide acrylique et de préférence est l'acide méthacrylique.

6. Composition selon l'une quelconque des revendications 1 à 5, **caractérisée en ce que** dans ledit copolymère, le monomère A est présent dans des quantités allant de 20 à 80% en poids et plus particulièrement de 25 à 70 % en poids et encore plus particulièrement de 35 à 60% en poids par rapport au poids total du copolymère.

7. Composition selon l'une quelconque des revendications 1 à 6, **caractérisée en ce que** dans ledit copolymère, le monomère B est présent dans des quantités allant de 15 à 80% en poids et plus particulièrement de 25 à 75 % en poids et encore plus particulièrement de 40 à 65% en poids par rapport au poids total du copolymère.

8. Composition selon l'une quelconque des revendications 1 à 7, où le copolymère réticulé est réticulé par au moins un agent réticulant éthylèniquement polyinsaturé.

9. Composition selon la revendication 8, **caractérisée en ce que** la teneur en agent réticulant varie de 0,01 à 5% en poids et de préférence de 0,03 à 3% en poids et encore plus particulièrement de 0,05 à 1% en poids par rapport au poids total du copolymère.

10. Composition selon l'une quelconque des revendications 1 à 9, **caractérisée en ce que** le copolymère réticulé est un copolymère réticulé d'acide méthacrylique et d'acrylate d'alkyle en C₁-C₄.

11. Composition selon l'une quelconque des revendications 1 à 10, **caractérisée en ce que** le copolymère réticulé est un copolymère réticulé d'acide méthacrylique et d'acrylate d'éthyle .

12. Composition selon l'une quelconque des revendications précédentes, **caractérisée en ce que** ledit copolymère réticulé est présent à une concentration comprise allant de 0,01 à 10% en poids par rapport au poids total de la composition et de préférence de 0,1 à 5% en poids.

13. Composition selon l'une quelconque des revendications 1 à 12, **caractérisé en ce** les alkylpolyglycosides sont représenté par la formule générale suivante :
R₁Q-(R₂O)ₜ (G)ᵥ
dans laquelle R₁ représente un radical alkyle et/ou alcényle linéaire ou ramifié comportant environ de 8 à 24 atomes de carbone, un radical alkylphényle dont le radical alkyle linéaire ou ramifié comporte de 8 à 24 atomes de carbone, R₂ représente un radical alkylène comportant environ de 2 à 4 atomes de carbone, G représente un sucre comportant de 5 à 6 atomes de carbone, t désigne une valeur allant de 0 à 10, de préférence 0 à 4, et v désigne une valeur allant de 1 à 15.

14. Composition selon l'une des revendications précédentes, **caractérisée en ce que** le ou les tensioactifs non ioniques sont présents dans les compositions selon l'invention dans des concentrations allant de 8 à 18 % en poids par rapport au poids total de la composition.

15. Composition selon l'une des revendications précédentes, **caractérisée en ce que** la composition comprend en outre des tensioactifs additionnels tels que des tensioactifs anioniques, amphotères, zwittérioniques et leurs mélanges.

16. Composition selon la revendication précédente, **caractérisée en ce que** la teneur en tensioactif(s) additionnel(s) va de 0,1 à 20% en poids, par rapport au poids total de la composition, plus particulièrement de 1 % à 10 % en poids, par rapport au poids total de la composition.

17. Composition selon l'une quelconque des revendications précédentes, **caractérisée en ce qu'**elle comprend en outre au moins un agent conditionneur.

18. Composition selon la revendication précédente, **caractérisée en ce que** le ou les agents conditionneurs sont choisis parmi les poly-α-oléfines, les huiles fluorées, les cires fluorées, les gommes fluorées, les esters d'acides carboxyliques, les silicones, les polymères cationiques, les huiles minérales, végétales ou animales, les céramides, les pseudocéramides, et leurs mélanges.

19. Composition selon l'une quelconque des revendications 17 ou 18, **caractérisé en ce que** le ou les agents conditionneurs sont choisis parmi les polymères cationiques et les silicones.

20. Composition selon la revendication précédente, **caractérisé en ce que** l'agent conditionneur est une silicone aminée.

21. Composition selon l'une quelconque des revendications 17-20, **caractérisée en ce** les agents conditionneurs représentent de 0,001 % à 10 % en poids, de préférence de 0,005 % à 5 % en poids, et encore plus préférentiellement de 0,01 % à 3 % en poids, du poids total de la composition.

22. Composition selon l'une quelconque des revendication précédente, **caractérisé en ce que** la composition est conditionnée dans un dispositif aérosol.

23. Utilisation d'une composition selon l'une quelconque des revendications précédentes pour la limitation du dégorgement et/ou l'amélioration de la protection de la coloration de fibres kératiniques.

24. Procédé de traitement cosmétique des matières kératiniques en particulier des cheveux, **caractérisé par le fait qu'**il consiste à appliquer sur lesdites matières une composition telle que définie selon l'une quelconque des revendications 1 à 22 puis à effectuer éventuellement un rinçage à l'eau.

## Claims

1. Detergent cosmetic composition comprising, in a physiologically acceptable aqueous medium, and in particular a cosmetically acceptable aqueous medium, from 5 to 20% by weight of a nonionic surfactant chosen from:
• alkylpolyglycosides;
and at least 0.01 to 10% by weight relative to the total weight of the composition of a crosslinked anionic copolymer of vinyl carboxylic acid (monomer A) and of a monomer having at least one α,β-ethylenic unsaturation (monomer B) chosen from the monomers of formulae:
i) CH₂=CXY
in which X denotes hydrogen and Y denotes -COOR,
-C₆H₄R', -CN, -CONH₂, -NHCOCH₃, -CONHC(CH₃)₃, -CON(CH₃)₂, or
X denotes CH₃ and Y denotes -COOR, -C₆H₄R', -CN or -CH=CH₂ ,
R denotes a C₁-C₄ alkyl or a C₂-C₄ hydroxyalkyl,
R' denotes hydrogen, a C₁-C₈ alkyl or a C₂-C₈ hydroxyalkyl.

2. Composition according to Claim 1, **characterized in that** the monomer B is chosen from methyl acrylate, ethyl acrylate, n-butyl acrylate, styrene, acrylamide, N,N-dimethylacrylamide, tert-butylacrylamide, methyl methacrylate, ethyl methacrylate, n-butyl methacrylate.

3. Composition according to the preceding claim, **characterized in that** the monomer B is ethyl acrylate.

4. Composition according to any one of Claims 1 to 3, **characterized in that** the monomer A is chosen from acrylic acid, methacrylic acid, itaconic acid, fumaric acid, crotonic acid, maleic acid and mixtures thereof.

5. Composition according to the preceding claim, **characterized in that** the monomer A is chosen from methacrylic acid and acrylic acid, and is preferably methacrylic acid.

6. Composition according to any one of Claims 1 to 5, **characterized in that**, in said copolymer, the monomer A is present in amounts ranging from 20 to 80% by weight, and more particularly from 25 to 70% by weight, and even more particularly from 35 to 60% by weight, relative to the total weight of the copolymer.

7. Composition according to any one of Claims 1 to 6, **characterized in that**, in said copolymer, the monomer B is present in amounts ranging from 15 to 80% by weight, and more particularly from 25 to 75% by weight, and even more particularly from 40 to 65% by weight, relative to the total weight of the copolymer.

8. Composition according to any one of Claims 1 to 7, where the crosslinked copolymer is crosslinked with at least one ethylenically polyunsaturated crosslinking agent.

9. Composition according to Claim 8, **characterized in that** the content of crosslinking agent ranges from 0.01 to 5% by weight, and preferably from 0.03 to 3% by weight, and even more particularly from 0.05 to 1% by weight, relative to the total weight of the copolymer.

10. Composition according to any one of Claims 1 to 9, **characterized in that** the crosslinked copolymer is a crosslinked copolymer of methacrylic acid and of C₁-C₄ alkyl acrylate.

11. Composition according to any one of Claims 1 to 10, **characterized in that** the crosslinked copolymer is a crosslinked copolymer of methacrylic acid and of ethyl acrylate.

12. Composition according to any one of the preceding claims, **characterized in that** said crosslinked copolymer is present at a concentration ranging from 0.01 to 10% by weight relative to the total weight of the composition, and preferably from 0.1 to 5% by weight.

13. Composition according to any one of Claims 1 to 12, **characterized in that** the alkylpolyglycosides are represented by the following general formula:
R₁O-(R₂O)ₜ(G)ᵥ
in which R₁ represents a linear or branched alkyl and/or alkenyl radical containing approximately from 8 to 24 carbon atoms, or an alkylphenyl radical in which the linear or branched alkyl radical contains from 8 to 24 carbon atoms, R₂ represents an alkylene radical containing approximately from 2 to 4 carbon atoms, G represents a sugar unit containing from 5 to 6 carbon atoms, t denotes a value ranging from 0 to 10, preferably from 0 to 4, and v denotes a value ranging from 1 to 15.

14. Composition according to one of the preceding claims, **characterized in that** the nonionic surfactant(s) is (are) present in the compositions according to the invention in concentrations ranging from 8 to 18% by weight relative to the total weight of the composition.

15. Composition according to one of the preceding claims, **characterized in that** the composition also comprises additional surfactants, such as anionic, amphoteric or zwitterionic surfactants, and mixtures thereof.

16. Composition according to the preceding claim, **characterized in that** the content of additional surfactant(s) ranges from 0.1 to 20% by weight, relative to the total weight of the composition, more particularly from 1% to 10% by weight, relative to the total weight of the composition.

17. Composition according to any one of the preceding claims, **characterized in that** it also comprises at least one conditioner.

18. Composition according to the preceding claim, **characterized in that** the conditioner(s) is (are) chosen from poly-α-olefins, fluoro oils, fluoro waxes, fluoro gums, carboxylic acid esters, silicones, cationic polymers, mineral, plant or animal oils, ceramides, pseudoceramides and mixtures thereof.

19. Composition according to either one of Claims 17 and 18, **characterized in that** the conditioner(s) is (are) chosen from cationic polymers and silicones.

20. Composition according to the preceding claim, **characterized in that** the conditioner is an aminosilicone.

21. Composition according to any one of Claims 17 to 20, **characterized in that** the conditioners represent from 0.001% to 10% by weight, preferably from 0.005% to 5% by weight, and even more preferably from 0.01% to 3% by weight, of the total weight of the composition.

22. Composition according to any one of the preceding claims, **characterized in that** the composition is packaged in an aerosol device.

23. Use of a composition according to any one of the preceding claims, for limiting the bleeding and/or improving the protection of the coloration of keratin fibres.

24. Process for cosmetically treating keratin substances, in particular the hair, **characterized in that** it consists in applying, to said substances, a composition as defined in any one of Claims 1 to 22, and then in optionally performing rinsing with water.

## Patentansprüche

1. Reinigende kosmetische Zusammensetzung, die in einem physiologisch akzeptablen und insbesondere kosmetisch akzeptablen, wässrigen Medium, bezogen auf das Gesamtgewicht der Zusammensetzung, 5 bis 20 Gew.-% eines nichtionischen grenzflächenaktiven Stoffes, der unter
■ den Alkylpolyglycosiden ausgewählt ist;
und mindestens 0,01 bis 10 Gew.-% eines vernetzten anionischen Copolymers einer Vinylcarbonsäure (Monomer A) und eines Monomers mit mindestens einer α,β-ethylenisch ungesättigten Bindung (Monomer B) enthält, die unter den Monomeren der folgenden Formeln ausgewählt sind:
i) CH₂=CXY
in der Formel:
X bedeutet Wasserstoff und Y bedeutet -COOR, -C₆H₄R', -CN, -CONH₂, -NHCOCH₃, -CONHC(CH₃)₃ oder -CON(CH₃)₂ oder X bedeutet -CH₃ und Y bedeutet -COOR, -C₆H₄R', -CN oder -CH=CH₂,
R bedeutet eine Alkylgruppe mit 1 bis 4 Kohlenstoffatomen oder eine Hydroxyalkylgruppe mit 2 bis 4 Kohlenstoffatomen;
R' bedeutet ein Wasserstoffatom, eine C₁₋₈-Alkylgruppe oder eine C₂₋₈-Hydroxyalkylgruppe.

2. Zusammensetzung nach Anspruch 1, **dadurch gekennzeichnet, dass** das Monomer B unter Methylacrylat, Ethylacrylat, n-Butylacrylat, Styrol, Acrylamid, N,N-Dimethylacrylamid, *tert-*Butylacrylamid, Methylmethacrylat, Ethylmethacrylat und n-Butylmethacrylat ausgewählt ist.

3. Zusammensetzung nach dem vorhergehenden Anspruch, **dadurch gekennzeichnet, dass** es sich bei dem Monomer B um das Ethylacrylat handelt.

4. Zusammensetzung nach einem der Ansprüche 1 bis 3, **dadurch gekennzeichnet, dass** das Monomer A unter Acrylsäure, Methacrylsäure, Itaconsäure, Fumarsäure, Crotonsäure, Maleinsäure und deren Gemischen ausgewählt ist.

5. Zusammensetzung nach dem vorhergehenden Anspruch, **dadurch gekennzeichnet, dass** das Monomer A unter Methacrylsäure und Acrylsäure und vorzugsweise Methacrylsäure ausgewählt ist.

6. Zusammensetzung nach einem der Ansprüche 1 bis 5, **dadurch gekennzeichnet, dass** das Monomer A in dem Copolymer in Mengenanteilen im Bereich von 20 bis 80 Gew.-%, insbesondere 25 bis 70 Gew.-% und ganz besonders bevorzugt 35 bis 60 Gew.-%, bezogen auf das Gesamtgewicht des Copolymers, enthalten ist.

7. Zusammensetzung nach einem der Ansprüche 1 bis 6, **dadurch gekennzeichnet, dass** das Monomer B in dem Copolymer in Mengenanteile im Bereich von 15 bis 80 Gew.-%, insbesondere 25 bis 75 Gew.-% und ganz besonders bevorzugt 40 bis 65 Gew.-%, bezogen auf das Gesamtgewicht des Copolymers, enthalten ist.

8. Zusammensetzung nach einem der Ansprüche 1 bis 7, wobei das vernetzte Copolymer mit mindestens einem mehrfach ethylenisch ungesättigten Vernetzungsmittel vernetzt ist.

9. Zusammensetzung nach Anspruch 8; **dadurch gekennzeichnet, dass** der Mengenanteil des Vernetzungsmittels im Bereich von 0,01 bis 5 Gew.-%, vorzugsweise 0,03 bis 3 Gew.-% und besonders bevorzugt 0,05 bis 1 Gew.-%, bezogen auf das Gesamtgewicht des Copolymers, liegt.

10. Zusammensetzung nach einem der Ansprüche 1 bis 9, **dadurch gekennzeichnet, dass** das vernetzte Copolymer ein vernetztes Copolymer von Methacrylsäure und einem C₁₋₄-Alkylacrylat ist.

11. Zusammensetzung nach einem der Ansprüche 1 bis 10, **dadurch gekennzeichnet, dass** das vernetzte Copolymer ein vernetztes Copolymer von Methacrylsäure und Ethylacrylat ist.

12. Zusammensetzung nach einem der vorhergehenden Ansprüche, **dadurch gekennzeichnet, dass** das vernetzte Copolymer in einer Konzentration von 0,01 bis 10 Gew.-%, bezogen auf das Gesamtgewicht der Zusammensetzung, und vorzugsweise 0,1 bis 5 Gew.-% enthalten ist.

13. Zusammensetzung nach einem der Ansprüche 1 bis 12, **dadurch gekennzeichnet, dass** die Alkylpolyglycoside durch die folgende allgemeine Formel dargestellt werden:
R₁O-(R₂O)ₜ(G)ᵥ
worin die Gruppe R₁ eine geradkettige oder verzweigte Alkyl- und/oder Alkenylgruppe, die etwa 8 bis 24 Kohlenstoffatome aufweist, oder eine Alkylphenylgruppe bedeutet, deren geradkettige oder verzweigte Alkylgruppe 8 bis 24 Kohlenstoffatome aufweist, R₂ eine Alkylengruppe ist, die etwa 2 bis 4 Kohlenstoffatome aufweist, G einen Zucker bedeutet, der 5 bis 6 Kohlenstoffatome aufweist, t einen Wert von 0 bis 10 und vorzugsweise 0 bis 4 hat und v einen Wert von 1 bis 15 aufweist.

14. Zusammensetzung nach einem der vorhergehenden Ansprüche, **dadurch gekennzeichnet, dass** der oder die nichtionischen grenzflächenaktiven Stoffe in den erfindungsgemäßen Zusammensetzungen in Konzentrationen im Bereich von 8 bis 18 Gew.-%, bezogen auf das Gesamtgewicht der Zusammensetzung, enthalten sind.

15. Zusammensetzung nach einem der vorhergehenden Ansprüche, **dadurch gekennzeichnet, dass** die Zusammensetzung ferner ergänzende grenzflächenaktive Stoffe enthält, wie anionische, amphotere oder zwitterionische grenzflächenaktive Stoffe und deren Gemische.

16. Zusammensetzung nach dem vorhergehenden Anspruch, **dadurch gekennzeichnet, dass** der Mengenanteil des oder der ergänzenden grenzflächenaktiven Stoffe(s) im Bereich von 0,1 bis 20 Gew.-%, bezogen auf das Gesamtgewicht der Zusammensetzung, und insbesondere 1 bis 10 Gew.-%, bezogen auf das Gesamtgewicht der Zusammensetzung, liegt.

17. Zusammensetzung nach einem der vorhergehenden Ansprüche, **dadurch gekennzeichnet, dass** sie ferner mindestens ein Konditioniermittel enthält.

18. Zusammensetzung nach dem vorhergehenden Anspruch, **dadurch gekennzeichnet, dass** das oder die Konditioniermittel unter den Poly-α-olefinen, fluorierten Ölen, fluorierten Wachsen, fluorierten Gummis, Estern von Carbonsäuren, Siliconen, kationischen Polymeren, Mineralölen, pflanzlichen Ölen und tierischen Ölen, Ceramiden, Pseudoceramiden und deren Gemischen ausgewählt sind.

19. Zusammensetzung nach einem der Ansprüche 17 oder 18, **dadurch gekennzeichnet, dass** das oder die Konditioniermittel unter den kationischen Polymeren und den Siliconen ausgewählt sind.

20. Zusammensetzung nach dem vorhergehenden Anspruch, **dadurch gekennzeichnet, dass** das Konditioniermittel ein aminiertes Silicon ist.

21. Zusammensetzung nach einem der Ansprüche 17 bis 20, **dadurch gekennzeichnet, dass** die Konditioniermittel 0,001 bis 10 Gew.-%, vorzugsweise 0,005 bis 5 Gew.-% und besonders bevorzugt 0,01 bis 3 Gew.-% des Gesamtgewichts der Zusammensetzung ausmachen.

22. Zusammensetzung nach einem der vorhergehenden Ansprüche, **dadurch gekennzeichnet, dass** die Zusammensetzung in einer Aerosolvorrichtung konfektioniert ist.

23. Verwendung einer Zusammensetzung nach einem der vorhergehenden Ansprüche, um das Ausschwitzen zu begrenzen und/oder den Farbschutz der Keratinfasern zu verbessern.

24. Verfahren zur kosmetischen Behandlung von Keratinsubstanzen und insbesondere Haaren, **dadurch gekennzeichnet, dass** es darin besteht, auf die Keratinsubstanzen eine Zusammensetzung, wie sie in einem der Ansprüche 1 bis 22 definiert ist, aufzutragen und anschließend gegebenenfalls mit Wasser zu spülen.
